Europäisches Patentamt

**European Patent Office**

Office européen des brevets

(11) Publication number: **0 202 808**
**A2**

(12) **EUROPEAN PATENT APPLICATION**

(21) Application number: **86303398.1**

(22) Date of filing: **06.05.86**

(51) Int. Cl.⁴: **A 61 F 5/44**
**A 61 B 19/02**

(30) Priority: **20.05.85 US 735902**

(43) Date of publication of application:
**26.11.86 Bulletin 86/48**

(84) Designated Contracting States:
**BE DE FR GB**

(71) Applicant: **BAXTER TRAVENOL LABORATORIES, INC.**
**One Baxter Parkway**
**Deerfield, IL 60015(US)**

(72) Inventor: **Richmond, Frank M.**
**315 S. Ridgelane Ave. No.2F**
**Harvard Illinois 60033(US)**

(72) Inventor: **Ostensen, Ralph**
**3241 Milwaukee**
**Northbrook Illinois 60072(US)**

(74) Representative: **MacGregor, Gordon et al,**
**ERIC POTTER & CLARKSON 14 Oxford Street**
**Nottingham, NG1 5BP(GB)**

(54) **Bacterial barrier.**

(57) The invention is a bacterial barrier for a container which includes a labyrinth gas passageway and at least one flapper valve in combination to prevent bacterial ingress into a container while allowing gas to pass from the container into the environment.

FIG. I

# BACTERIAL BARRIER

## BACKGROUND OF THE INVENTION

This invention relates generally to bacterial barriers and relates more specifically to barriers which inhibit bacterial ingress from the environment into an enclosure while allowing gases to vent from the enclosure into the environment.

Those concerned with maintaining sterility in an enclosure for long periods of time frequently are faced with the need to allow gases to vent from the enclosure into the environment without allowing bacteria to enter the enclosure through a gas vent. A typical situation in which this need arises is in the urinary drainage field. One of the needs of a urinary drainage bag is to be able to vent gases which build up inside the bag as urine is collected to the environment without allowing bacteria from the environment to enter into the bag. If bacteria is allowed to enter into the bag, a urinary infection in a patient may result which is highly undesirable.

Previous attempts to provide air venting of urinary drainage bags include the provision of a labyrinth seal which allows air to vent from the urinary drainage bag while inhibiting the passage of bacteria into the bag. One reference describing such a labyrinth seal is U.S. Patent No. 4,192,295 issued to Sherlock on March 11, 1980. FIG. 1 of this patent illustrates a labyrinth seal 64 providing an air outlet at a top portion of a urinary drainage bag. While the labyrinth seal does tend to inhibit the ingress of bacteria into a urinary bag, such a seal has been found to.be not entirely successful in preventing bacterial ingress over periods of more than four to six days. Since some patients require long-term urinary drainage, a more successful type of bacterial barrier is desirable.

In other applications, it is desirable to provide a bacterial barrier for pouches containing materials to be sterilized. These materials may be medical materials, individual components, or very large assemblies. Regardless of the type of matter to be sterilized, if the matter is to be sterilized in a pouch using ethylene-oxide or heat sterilization techniques , it is necessary to provide some means of allowing gases to escape from the pouch during sterilization or the pouch may expand to the point of rupture. Thus, it is desirable to provide a simple, inexpensive form of gas venting for such pouches.

Other methods of providing gas venting which inhibits bacterial ingress includes porous plugs and filters. Materials such as high density polyethylene and polypropylene can be used as plugs. Almost any material can be used as a filter material provided that it has the required porosity and strength needed for a particular application.

## SUMMARY OF THE INVENTION

A microbial barrier for the prevention of ingress of bacteria from the environment into an inner container having a higher or equal gas pressure than the environment is described. The barrier includes first and second flexible sheets which are sealed together to form a labyrinth gas passageway. The gas passageway has an inlet and outlet. The barrier further includes at least one "flapper valve" formed from the first and second flexible sheets and contiguous with the outlet of the air passageway. The first and second flexible sheets are collapsible on each other in the valve so as to allow the passage of air through the valve from an inner container into the environment, but to prevent the passage of bacteria from the environment into the inner container.

In view of the foregoing, it is an object of the invention to provide a bacterial barrier which allows gases to flow in a first direction through the barrier and inhibits bacterial ingress in a second direction through the barrier.

It is another object of the invention to provide a low-cost bacterial barrier for a variety of applications including urinary drainage bags and sterilizable supply pouches.

Other objects, advantages, and novel features of the present invention will become apparent from the following detailed description of the invention when considered in conjunction with the accompanying drawings. Before explaining the embodiments of the invention in detail, it is to be understood that the invention is not limited in its application to the details of the construction and to the arrangement of components as set forth in the following description, or as illustrated in the accompanying drawings. The invention is capable of other embodiments and of being practiced and carried out in various ways. Furthermore, it is to be understood that the phraseology and terminology employed are for the purpose of description and should not be regarded as limiting.

## BRIEF DESCRIPTION OF THE DRAWINGS

FIG. 1 is a front view of a urinary drainage bag illustrating one embodiment of the subject invention; and

FIG. 2 is a front view of a medical supply pouch illustrating another embodiment of the subject invention.

## DETAILED DESCRIPTION OF THE PREFERRED EMBODIMENT

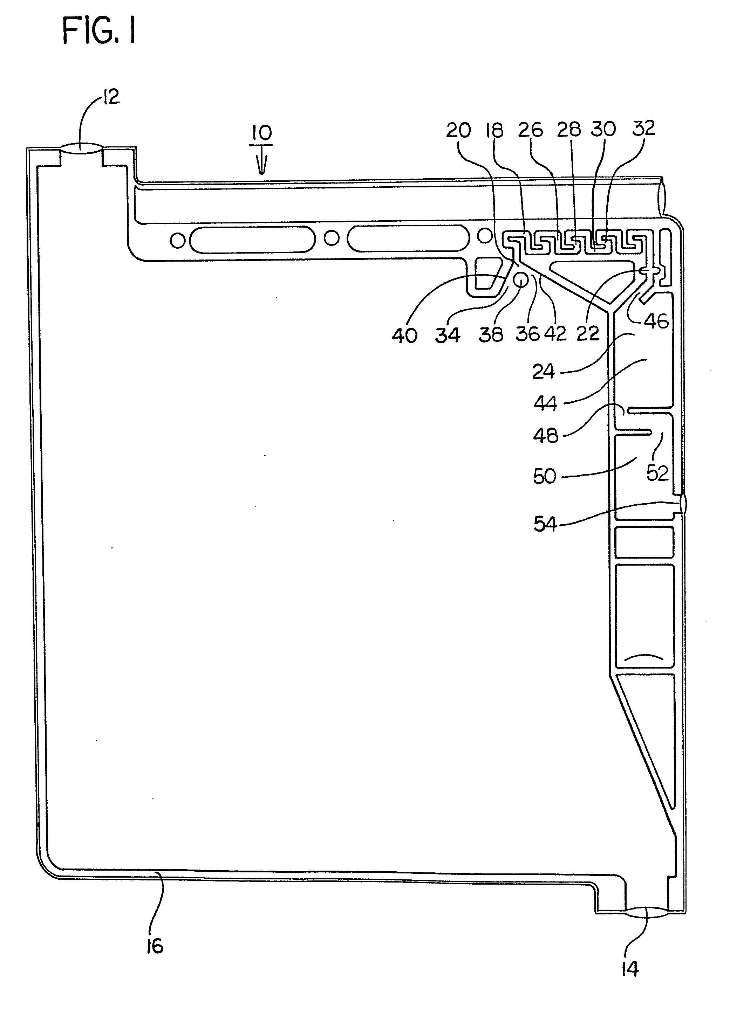

Refer now to FIG. 1 which illustrates one embodiment of the subject invention. In this embodiment, a urinary drainage bag 10 has an inlet port 12 to receive urine and an outlet port 14 to drain urine from the bag 10. The urinary drainage bag 10 is formed of first and second layers of flexible material which are sealed 16 substantially about the periphery. The seal forms a labyrinth gas passageway 18 which allows gases which build up in the bag 10 to be vented from the bag. In the preferred embodiment of the subject invention, the passageway 18 includes an inlet 20 to receive gases from the bag 10. The gas passageway includes an outlet 22 which is contiguous with a flapper valve 24, discussed in greater detail below. The labyrinth gas passageway includes both horizontal and vertical flow segments, the horizontal flow segments include both forward-flow and backward-flow segments and the vertical flow segments also include both upward-flow, and downward-flow portions to cause gas passing through the passageway to be forced to flow in four different directions sequentially; thus, gas passing through the labyrinth passageway is caused to flow in opposite directions, both horizontally and vertically.

This can be more clearly understood with reference to FIG.1 which illustrates that the labyrinth passageway includes both a downward segment 26 and an upward segment 28 which causes the gas to flow in opposite directions vertically. The labyrinth passageway also includes a forward section 30 and a backward section 32 which causes the gas to flow in opposite directions horizontally. The unique feature of the labyrinth gas passageway in the preferred embodiment is that it requires air to flow in opposite directions, both vertically and horizontally. This distinguishes the subject labyrinth passageway over the passageway described in U.S. Patent No. 4,192,295 because the passageway described therein only requires gas to flow in opposite directions in the vertical plane only.

Another feature of the preferred embodiment related to the labyrinth passageway includes the fact that the inlet 20 of the labyrinth passageway is composed of first and second gas passages 34 and 36 respectively which are formed by locating a disc-shaped seal 38 in close proximity to inlet 20 of the labyrinth passageway. The inlet of the gas passageway in the preferred embodiment forms two sides 40, 42 of a delta with the seal 38 located between two sides of the delta. The angle formed by each side of the delta is such that if the urinary bag is folded so as to collapse, one of the first or second passages 34, 36, the other of the passages will remain open to allow gas to escape. Furthermore, this configuration has been found to prevent urine from blocking the inlet to the gas passageway. It should be recognized that in other embodiments, other shapes may be substituted for the disc-shaped seal to provide a means for maintaining the inlet open.

In the preferred embodiment, a "flapper" valve 24 is disposed at the outlet 20 of the labyrinth passageway. A flapper valve is a valve that is formed from two layers of flexible material that are collapsible on each other to cause the valve to remain shut when the pressure on the outlet side of the valve is greater than or equal to the pressure on the inlet side of the valve. Thus, the valve will remain substantially closed in the absence of gas pressure inside the urinary bag which would cause the flapper valve to open. The valve would then remain open only until the gas pressure inside the urinary bag has been sufficiently reduced to cause the two sides of the flexible material to collapse on each other again. Thus, whenever the flapper valve is open, gas will be flowing from the urinary drainage bag to the environment. This flow of gas prevents bacteria from ingressing into the urinary drainage bag while the flapper bag is open. Once the air pressure from the gas has been reduced and the flapper valve has closed, bacteria is prevented from entering into the urinary bag due to the collapsed layers of material.

In the preferred embodiment, a first flapper valve 44 is formed from the first and second flexible sheets contiguous with the outlet 20 of the labyrinth gas passageway, the first valve 44 includes an inlet 46 and an outlet 48, the first and second sheets are collapsible on each other in the first valve 44. The preferred embodiment also includes a second flapper valve 50 formed from the first and second flexible sheets, the second flapper valve 50 has an inlet 52 contiguous with the outlet 48 of the first valve 44.

The labyrinth passageway is a small, very thin space which prohibits the movement of bacteria therethru. It is desired to prohibit passage of rod bacteria in excess of 0.2 microns in the examples described. The size and shape of the labryinth determines its effectiveness. However, its efficiency can be further enhanced if both its portal inlet and outlet openings are pre-disposed closed by the addition of means which are referred to as "flapper valves". The weldment illustrated as element 38 accomplishes this at the inlet even though liquid in the bag wants to pull the sheets apart, further exposing the portal 20. It perhaps should be pointed out that element 38 also inhibits wetting portal 28 with urine, which could block the passageway, rendering it ineffective.

The inside of urinary drainage bags should be at or close to atmospheric pressure for optimum functioning and patient safety. If drainage system is not vented, a negative pressure can result in the bag until the bag is approximately half-full. Gas may flow from the environment into the bag to eliminate such negative pressure. It is possible for gasses to flow from the environment into the bag through a flapper valve in some embodiments if at least one layer of material forming the flapper valve is formed of a roughened or textured material to produce a plurality of submicron or micron-sized passageways.

It should be noted that in the preferred embodiment of the subject invention, the outlet 54 of the second valve is disposed on a periphery of the drainage bag that is not facing upwardly when the drainage bag is in normal use. This feature reduces the possibility of the ingress of bacteria by gravitational forces into the drainage bag.

## EXPERIMENTAL RESULTS

The combination of a flapper valve and a labyrinth passageway has resulted in unexpected results relating to inhibiting bacterial ingress into an enclosure such as a urinary drainage bag. The combination of the flapper valve and labyrinth passageway were tested against a simple labyrinth passageway according to the following procedure.

Test Number 1 - Standard Labyrinth Passageway Design:

Seventy two urinary drainage bags having a labyrinth passageway similar to the labyrinth passageway described in U.S. Patent No. 4,192,295 were sterilized using typical ethylene oxide sterilization techniques. Ten additional bags had the air vents sealed so that the bags could act as negative controls during the test. Ten other bags were injected with a standardized suspension of the Serratia marcescens test organisms. All of the drainage bags were then aseptically filled with 500 ml of sterile soybean casein digest (SCD) broth, sealed and incubated at 30-35°C for 4 days. Prior to testing the bags were examined for contamination growth and none was observed.

An overnight SCD culture of Serratia marcescens was turbidimetrically adjusted to 29% transmittance at 650 nm. A 1:10 dilution of the adjusted culture was performed in gel phosphate buffer to yield a concentration of approximately $1 \times 10^8$ organisms per ml to be used as the aerosol challenge suspension in the nebulizer.

The $10^{-6}$ through $10^{-8}$ dilutions of the adjusted culture were plated in triplicate to verify the actual suspension population.

Testing was performed in a laminar flow-hood. The nebulizer was charged with the challenge suspension, situated on the ring stand in the hood and connected to the nitrogen source set at a pressure of 4 psi. The nebulizer aerosol was examined for proper operation prior to beginning the testing.

Each test unit was hung in the test hood with the torturous path air vent situated approximately 4 inches from the mouth of the nebulizer. The drain tube of the bag extended outside the hood and into a sterile container to collect the SCD broth that was drained out during testing. Each unit was tested separately by exposing it to the aerosolized challenge suspension for approximately one minute while 250-350 ml of SCD broth was simultaneously drained out of the bag. This test procedure was designed to simulate in-use conditions of the air vent. After exposure the drainage tube was sealed and the bags incubated at 30-35°C for 6 days.

The negative controls were tested in the same manner as the test units except the opening of the air vent was sealed prior to testing. The positive control units were tested in the same manner as the test units except the front sheeting of the bag near the air vent had been comprised with a sterile needle prior to testing.

Three negative and positive control units were tested at the beginning of the testing procedure four of each in the middle, and three of each at the end to insure proper operation of the test system during the course of the procedure.

Following incubation, each bag was examined for microbial growth in the SCD broth. Any test units exhibiting growth were subcultured onto SCD agar plates; ornithine and oxidase tests and gram stains were performed to confirm the presence of the Serratia marcescens challenge organism.

Results Test #1:

The results of the aerosol integrith challenge of the torturous path (labyrinth seal) gas vent are as follows:

|  | No. Units Positive | No. Units Tested |
|---|---|---|
| Test Units | 3 | 72 |
| Negative Controls | 0 | 10 |
| Positive Controls | 10 | 10 |

The population of the challenge suspension used in the nebulizer was $5.4 \times 10^7$ organisms per ml. The three positive units were confirmed as the challenge organism by means of the gram stains and ornithine and oxidase test results.

The results of this test indicate that the challenge organism was able to penetrate this particular design of torturous path gas vent. This test results in a 48% confidence level that the true contamination rate (or failure rate) is less than or equal to 0.05.

## Test Number 2 - Back-Flow Labyrinth Passage In Combination with Flapper Valves

In two other tests, urinary drainage bags having gas vent seals of the type illustrated in FIG. 1 were tested. Both of these tests revealed that the gas vent was 100% effective as a microbial barrier. In one of the tests, 79 bags were tested using the same procedures described above. The results of this test are set forth below.

## Results Test #2:

The results of the aerosol integrity challenge of the second generation design tortuous path (labyrinth seal) gas vent were as follows.

| Test and Control Articles | No. of Units Positive | No. of Units Tested |
|---|---|---|
| Test Units | 0 | 59 |
| Negative Controls | 0 | 10 |
| Positive Controls | 10 | 10 |

The test and control articles were exposed to a nebulized challenge suspension of Serratia marcescens at a population of $1.5 \times 10^8$ organisms/ml for approximately 1 minute and incubated at 30°C for 5 days. The number of units positive were determined by visual inspection

Results Test #3:

In the third test, 69 bags (59 test articles, 5 negative controls and 5 positive controls) were tested using the same procedures described above. The results of the aerosol integrith challenge of the modified second generation design tortuous path (labyrinth seal) gas vent are provided in Table 1.

| Test and Control Articles | No. of Units Positive | No. of Units Tested |
|---|---|---|
| Test Units | 0 | 59 |
| Negative Controls | 0 | 5 |
| Positive Controls | 5 | 5 |

The test and control articles were exposed to a nebulized challenge suspension of Serratia marcescens at a population of $1.06 \times 10^8$ organisms/ml for approximately 1 minute and incubated at 30°C to 35°C for approximately 6-1/2 days. The number of units that were positive was determined by visual inspection and confirmed by colonial growth, chemical tests, and microscopic evaluation.

ALTERNATIVE EMBODIMENTS

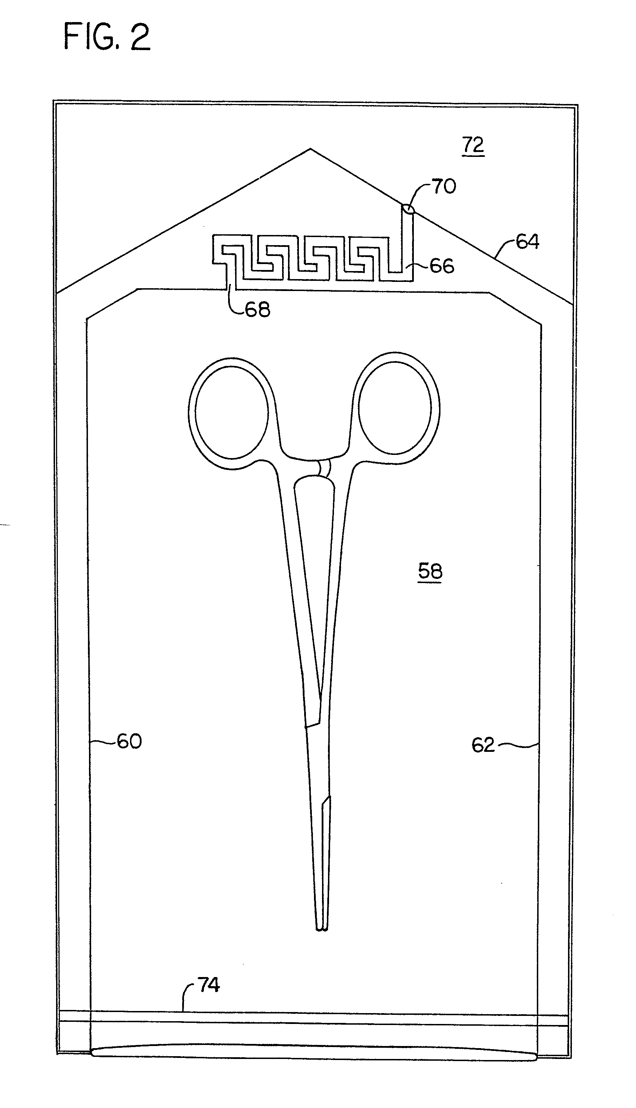

Another alternative embodiment of the subject invention is illustrated in FIG. 2. In this Figure, a pouch 58 is formed of two layers of flexible material. The periphery of the pouch is sealed on two opposing sides 60, 62 during the manufacture of the pouch. A third side 64 is provided with a labyrinth passageway 66, having an inlet 68 connecting the passageway to the inside of the pouch 58. The passageway 66 also includes an outlet 70 which is connected to a flapper valve 72. The flapper valve 72 is formed simply from the two layers of flexible material at one end of the pouch. The opposite end of the pouch is allowed to remain open during manufacture of the pouch in one embodiment of the subject invention. This type of pouch is useful in medical applications in which it is desired to insert a medical instrument or other component through an opening 74 at a later time and then seal the opening 74. The medical instrument can then be sterilized using ethylene-oxide sterilization techniques or other forms of sterilization techniques such as autoclaving techniques. The combination of the labyrinth passageway 66 and flapper valve 72 at the opposite end of the pouch, allows gases to escape during the sterilization process. This combination, however, prevents bacteria from entering the pouch at a later date as the medical device is stored. This combination is particularly useful in ethylene-oxide sterilization techniques because it provides for a method of venting the ethylene-oxide gas residuals fairly rapidly so that containers sterilized using this technique do need to be stored for long periods of time simply for the purpose of allowing the ethylene-oxide gas residual to dissipate to a desired level.

It should be recognized that the combination of a labyrinth seal and a flapper valve can be used in a wide variety of applications to allow gases to vent from a container while preventing bacteria from entering the container from the environment. This combination can be useful in any type of packaging where this combination of features is desired.

Although the invention has been described and illustrated in detail, it is to be clearly understood that the same is by way of illustration and example only, and is not to be taken by way of limitation; the spirit and scope of this invention being limited only by the terms of the appended claims.

THAT WHICH IS CLAIMED;

Claim 1. A microbial barrier for the prevention of ingress of bacteria from the environment into an inner container comprising:

first and second flexible sheets, said sheets being sealed to form a labyrinth gas passageway, said gas passageway having an inlet and outlet; and

a first valve formed from said first and second flexible sheets, said first valve including an inlet and an outlet, said inlet of said first valve being contiguous with said outlet of said labyrinth gas passageway, said first and second sheets being collapsible on each other in said first valve so as to allow the passage of gas through said valve, but to prevent the passage of bacteria from the environment into the inner container through said first valve.

Claim 2. A microbial barrier as recited in Claim 1, further comprising:

a second valve formed from said first and second flexible sheets, said second valve having an inlet contiguous with said outlet of said first valve, said first and second sheets being collapsible on each other in said second valve so as to allow the passage of gas through said barrier from said first valve through said second valve to the environment, but to prevent the passage of bacteria from the environment to the first valve through said second valve.

Claim 3. A microbial barrier as recited in Claim 1, wherein:

said inner container is a urinary drainage bag.

Claim 4. A microbial barrier as recited in Claim 1, wherein:

said inner container is a pouch for containing sterilizable medical instruments.

Claim 5. A microbial barrier as recited in Claim 1, wherein:

said inner container is a pouch for containing sterilizable individual components.

Claim 6. A microbial barrier as recited in Claim 1, wherein:

said inner container is a pouch for containing sterilizable assemblies.

Claim 7. A microbial barrier as recited in Claim 3, wherein:

said outlet of said second valve is disposed on periphery of said drainage bag that is not facing upwardly when said drainage bag is in normal use.

Claim 8. An enclosure for containing sterile matter, comprising:

first and second flexible sheets, said sheets having a seal to form a valve, said valve having gas vent means for allowing gas in said enclosure to pass out of said enclosure to the environment while inhibiting passage of bacteria from the environment through said gas vent means into said enclosure, said gas vent means including a labyrinth gas passageway having an inlet contiguous with said enclosure, said labyrinth gas passageway also having an outlet, said gas vent means also having a flapper valve having an inlet contiguous with said outlet of said labyrinth gas passageway, said flapper valve having an outlet to the environment, said first and second sheets being collapsible on each other in said flapper valve to allow gas to flow from said enclosure to the environment and to inhibit bacterial ingress from the environment into said enclosure.

Claim 9. An enclosure as recited in Claim 8, wherein: said flapper valve includes at least one extending portion to prevent air from flowing in a straight line from said inlet of said flapper valve to said outlet of said flapper valve.

Claim 10. An enclosure as recited in Claim 8, wherein: said seal of said first and second flexible sheets at said inlet of said labyrinth passageway forms two sides of a delta, said seal also having a disc-shaped portion located in close proximity to said inlet of said labyrinth passageway to form first and second passageways from said enlclosure to said passageway.

FIG. 1

0202808

FIG. 2